# EUROPEAN PATENT SPECIFICATION

(11) **EP 2 779 946 B1**
(45) Date of publication and mention of the grant of the patent: **02.08.2017**
(21) Application number: 12850392.7
(22) Date of filing: 14.11.2012
(51) Int. Cl.: A61F 2/38

(54) **KNEE REVISION PROSTHESIS WITH PROGRESSIVE RESTRAINT**
REVISIONSPROTHESE FÜR DAS KNIE MIT PROGRESSIVER RÜCKHALTUNG
PROTHÈSE DE RÉVISION DU GENOU AVEC RETENUE PROGRESSIVE

(30) Priority: 14.11.2011 US 201113296220
(43) Date of publication of application: 24.09.2014
(73) Proprietor: Maxx Orthopedics, Inc., Plymouth Meeting, PA 19462 (US)
(72) Inventor: SHAH, Asit, Ridgewood, NJ 07450 (US); PERINE, Corey, Rydal, PA 19046 (US)
(74) Representative: Dunleavy, Kevin James
(86) International application number: PCT/US2012/065121
(87) International publication number: WO 2013/074700

(56) References cited:
- GB-A- 2 362 325
- US-A- 5 147 405
- US-A1- 2009 204 221
- US-A1- 2009 319 048
- US-A1- 2009 319 048
- US-A1- 2011 125 275
- US-A1- 2011 125 275
- US-A1- 2011 125 279
- US-B2- 6 475 241

## Description

### Field of the Invention

The present invention relates to a revision knee prosthesis that provides decreasing coronal and transverse rotational constraint as the knee rotates from extension to flexion, and then increasing constraint as the knee rotates from flexion to full extension.

U.S. Patent no. 5,147,405 relates to a knee prosthesis including a tibal component having first and second generally concave bearing surfaces and a stabilizing post extending upwardly between the first and second bearing surfaces. The knee prosthesis also includes a femoral component having first and second spaced apart, convexly curved condyle bearing surfaces and a cam surface extending between the first and second bearing surfaces. The femoral component is articulatable with the tibial component between an extended position and a flexed position. The first and second femoral bearing surfaces engage the first and second tibial bearing surfaces, respectively, at first and second contact points. Between the extended position and about a 25° flexion angle, the cam surface is spaced apart from a posterior side wall of the stabilizing post to permit movement of the femoral and tibial component as natural physiology dictates. As the knee prosthesis is flexed at about a 25° flexion angle, the cam surface first engages the posterior side wall of the stabilizing post to control movement of the femoral component relative to the tibial component. Therefore, the stabilizing post engages the cam surface to control the position of the femoral component relative to the tibial component only upon flexion of the femoral and tibial component between about 25° flexion and the flexed position.

US 2011/125275 A1 discloses a prosthetic joint that includes a first implant component for attachment to a first bone and a second implant component for attachment to a second bone. The first implant component has a condylar portion that includes first and second condylar bearing surfaces and similarly, the second implant component has bearing surfaces that receive and are complementary to the first and second condylar bearing surfaces. Each of the first and second condylar bearing surfaces and each of the bearing surfaces of the second implant component has a cross-section in a coronal plane that exhibits two different radii and a contact point is established between the first and second condylar bearing surfaces and the bearing surfaces of the second implant component. The bearing surfaces of the respective implant components are configured such that varus and valgus rotation of the first implant component relative to the second implant component causes the contact point to move outwardly.

GB 2 362 325 A discloses a condylar total kneed replacement prosthesis having interacting guide surfaces for control of anterior-posterior displacement. The prosthesis comprises a femoral component having a pair of condylar surfaces, a tibial component having a tibial platform and a bearing component interposed between the femoral and tibial components. A femoral guide surface is located between the condyles and engages with a tibial guide surface to cause the femoral component to displace posteriorly during flexing movements and displace anteriorly during extending movements. The bearing component is a one-piece plastic member which may be mobile in an anterio-posterior direction on the tibial platform. Knee revision surgery, also known as revision total knee arthroplasty, is a procedure in which the surgeon removes a previously implanted artificial knee joint, or "primary" prosthesis, and replaces it with a new "revision" prosthesis. Replacement of the "primary" prosthesis is required when it becomes defective or its connection to the femur and/or tibia becomes compromised (loosened), both of which may occur for several reasons. For example, the primary prosthesis may simply wear out or be damaged from patient trauma. However, the most common condition requiring primary prosthesis replacement is weakening or degradation of the anatomy to which the primary prosthesis is attached, which is commonly caused by osteolysis. Osteolysis is an inflammatory response that occurs when tiny fragments of polymer liner are absorbed by tissue cells around the knee joint. The inflammatory response dissolves the bone around the prosthesis and eventually loosens its connection thereto. In such case, it is usually not possible to surgically re-attach the primary prosthesis since the bone has typically degraded to a condition that is incompatible for reconnection to a primary device. Furthermore, the patient's bone is further degraded when the primary prosthesis, which is typically cemented to the bone, is removed by chipping away at the bone.

While the articulating surfaces of a revision prosthesis are similar to a primary prosthesis, the design of a revision prosthesis accommodates the fact that there is typically less bone to which the prosthesis is attached. For example, a revision prosthesis typically includes integrated stems that are inserted into the intermedullary canal of the femur and tibia for better support and stability. A revision prosthesis may also include attachable augments of varying thicknesses connected to the tibial base plate or the femoral component to accommodate irregularities in the bone profile. These components enable the bone to better support the device.

In addition to providing greater support on the bones, the design of a revision prosthesis typically provides greater constraint than a primary prosthesis. As the knee is flexed, the most obvious rotation of the tibia occurs in the sagittal plane about a coronal axis relative to the femur. However, the femur also translates posteriorly on the tibia and the tibia also rotates internally about its longitudinal axis. The complex articulation path of the human knee is dictated by the geometry of the distal femur and proximal tibia, as well as the arrangement of ligaments surrounding and connecting the distal femur and proximal tibia. The collateral ligaments provide stability to the knee in varus and valgus stresses. The cruciate ligaments provide stability in the anterior and posterior direction, and also allow the tibia to rotate axially, *i.e.,* about its longitudinal axis. Thus, as the knee is flexed, the tibia undergoes internal rotation about its longitudinal axis.

During primary knee replacement surgery, the anterior cruciate ligament (ACL), and sometimes posterior cruciate ligament (PCL), is removed but the collateral ligaments remain intact. During revision knee surgery, the PCL (if remaining) and the collateral ligaments are almost always removed. Therefore, the stability/constraint that was previously provided by these ligaments must be provided by the design of the revision prosthesis itself.

To provide stability, prior art revision prostheses include a femoral component with a guide box and a tibial liner with a central a central post, which articulates within the guide box. The post and guide box of prior art revision prostheses are designed and dimensioned to allow free rotation of the tibia in the sagittal plane but little rotation in the transverse or coronal plane. Any rotation in the transverse or coronal plane is due to the clearance or laxity between the engaging surfaces of the post and guide box, which enables some movement in both the coronal and transverse planes.

In the prior art, the amount of clearance intentionally designed between the post and the guide box is dictated by two competing considerations based on the natural articulation of the knee. To mimic the complex articulation path of the human knee, a revision prosthesis should provide high restraint during full extension but provide some laxity during flexion. Some prior art revision prostheses have very little clearance between the post and the guide box, which provides the desirable high level of constraint in full extension but undesirable high level of constraint during flexion. On the other hand, other prior art revision prostheses have greater clearance between the post and the guide box, which provides undesirable laxity in full extension but desirable laxity during flexion. It is believed, however, that none of the prior art revision prostheses provide high restraint during full extension and reduced restraint during flexion. Therefore, it would be desirable to provide a revision prosthesis, which more accurately facilitates the motion of the natural knee by providing a high degree of restraint during extension and less restraint during flexion.

### Summary of the Invention

The invention is defined in claim 1 and comprises a revision knee prosthesis that provides variable restraint as the knee rotates through its normal range of motion. In a first embodiment, the revision knee replacement prosthesis provides decreased restraint (increased laxity) as the knee rotates from full extension to flexion, and then provides increased restraint (decreased laxity) as the knee rotates from flexion to full extension. In a preferred embodiment, variation in restraint relates to valgus-varus laxity (rotation or tilt in the coronal plane) and/or medial-lateral laxity (rotation in the transverse plane).

The novel prosthesis comprises a femoral component that articulates with a tibial component and the natural or prosthetic patella. In a first embodiment, the revision knee prosthesis comprises a femoral component that connects to the distal end of a resected femur and a tibial component that connects to the proximal end of a resected tibia. The femoral component includes medial and lateral condyles having distal, articulating condylar surfaces, and a patellar flange having an articulating patellar surface. The tibial component includes a proximal bearing surface with medial and lateral concavities that articulate with the medial and lateral condyles. The condylar surfaces and concavities enable anterior-posterior translation of the femur relative to the tibia, and enable the tibia to rotate about its longitudinal axis during flexion of the knee. The prosthesis includes progressive constraint means for providing progressively increasing and progressively decreasing rotational constraint of the femoral component (20) in the coronal and transverse planes as the femoral component (20) rotates in the sagittal plane between full extension, to a position in flexion, and then back to full extension. The progressive constraint means provides rotational constraint of the femoral component (20) in the coronal and transverse planes at a non-constant rate of change as the femoral component (20) rotates in the sagittal plane between full extension, to a position in flexion, and then back to full extension.

In an embodiment, the femoral component includes a guide box fixed to the femoral mounting surfaces intermediate the condyles. The tibial component includes a central post fixed intermediate the tibial concavities, which articulates within the guide box and constrains rotational movement of the femoral and tibial components relative to one another in the coronal plane and transverse planes. The post and guide box are constructed and arranged to provide progressively decreasing and then progressively increasing rotational restraint of the femoral component in the coronal and transverse planes as the femoral component rotates in the sagittal plane between full extension to a position in flexion, and then back to full extension, respectively. In a preferred embodiment, the femoral component is restrained from rotating or tilting more than about 2 degrees in the coronal plane when the prosthesis is positioned in full extension and from rotating or tilting more than about 7 degrees in the coronal plane when the prosthesis is positioned in full flexion.
In a preferred embodiment, the femoral component is preferably restrained from rotating more than about 1 degree in the transverse plane when the prosthesis is positioned in full extension and from rotating more than about 4 degrees in the transverse plane when the prosthesis is positioned in full flexion.

In one embodiment, the change in restraint δR on the femoral component is variable over the full range of flexion. In another embodiment, the change in restraint δR on the femoral component has a first profile over a first range of flexion, and has a second profile over a second range of flexion.

The guide box has opposed side walls with interior surfaces, and the post has opposed exterior surfaces that engage the interior surfaces of the guide box. The guide box interior surfaces have a minimum interior width between them at an anterior end (AW) and a maximum interior width at a posterior end (PW), and the post has exterior surfaces with a constant width (CW) that is less than AW. In one embodiment the guide box side walls have exterior surfaces that are parallel to one another and interior side walls that are skew. The interior surfaces may have a planar surface or an irregular surface profile. In another embodiment, a cam is fixed to each of the interior surfaces of the side walls. The cams have interior surfaces that engage the exterior surfaces of the post. The interior cam surfaces have a minimum interior width between them at an anterior end (AW) and a maximum interior width at a posterior end (PW). The post has exterior surfaces have a constant width (CW) between them that is less than AW. The interior surfaces of the cams may also have a planar or an irregular surface profile.

The femoral component may include a cam connecting the posterior ends of the condyles. Anterior and posterior translation of the femoral component relative to the tibial component is controlled by the cam and central post. Rotation of the tibia about its longitudinal axis is also controlled, at least in part, by the cam and central post. Posterior translation caused by the cam and post is about 1-2 millimeters after the prosthesis is fully flexed.

### Brief Description of the Drawings

Fig. 1 is a perspective view of a knee prosthesis in full flexion;
Fig. 2 is a perspective view showing the proximal mounting surface of the femoral component shown in Fig. 1;
Fig. 3 is another perspective view showing the showing the proximal mounting surface of the femoral component shown in Fig. 1;
Fig. 4 is a top (proximal) plan view of the femoral component shown in Fig. 1;
Fig. 5 is a posterior elevation of the femoral component shown in Fig. 1;
Fig. 6 is an anterior elevation of the femoral component shown in Fig. 1;
Fig. 7 is a lateral elevation of the femoral component shown in Fig. 1;
Fig. 8 is a medial elevation of the femoral component shown in Fig. 1;
Fig. 9 is section view taken along lines 9-9 of Fig. 5;
Fig. 10 is an illustration (not to scale) of the guide box slot shown in Fig. 9;
Figs. 11 and 12 are anterior and posterior, respectively, perspective views of the tibial liner shown in Fig. 1;
Fig. 13 is a medial elevation of the tibial liner shown in Fig. 1;
Fig 14 is an anterior elevation of the tibial liner shown in Fig. 1;
Fig. 15 is a top (proximal) plan view of the tibial liner shown in Fig. 1;
Fig. 16 is a cross section taken along lines 16-16 of Fig. 15;
Fig. 17 is a cross section taken along lines 17-17 of Fig. 15;
Figs. 18a- 20c are illustrations of the progressively decreasing medial-lateral (transverse plane) rotational restraint of the femoral component as the femoral component rotates from full extension to an intermediate position, and then rotates to a full flexion;
Figs. 21a-23c are illustrations of the progressively decreasing valgus-varus (coronal plane) rotational laxity of the femoral component as the femoral component rotates from full extension to an intermediate position, and then rotates to a full flexion;
Fig. 24 is an illustration (not to scale) of the guide box slot not in accordance with the present invention;
Fig. 25 is an illustration (not to scale) of the guide box slot in accordance with the present invention; and,
Fig. 26 is an illustration (not to scale) of an alternative guide box slot not in accordance with the present invention.

### Detailed Description of Preferred Embodiments

For the purpose of illustrating the invention, several embodiments of the invention are shown in the accompanying drawings. However, it should be understood by those of ordinary skill in the art that the invention is not limited to the precise arrangements and instrumentalities shown therein and described below. Throughout the specification, like reference numerals are used to designate like elements. Numerous changes and modifications within the scope of the invention will become apparent to those skilled in the art from this detailed description.

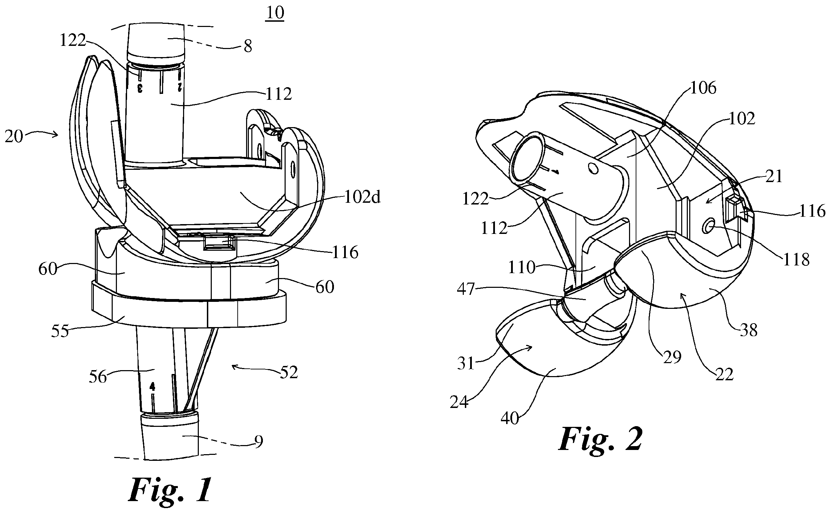

Unless otherwise defined, all technical and scientific terms used herein in their various grammatical forms have the same meaning as commonly understood by one of ordinary skill in the art to which this invention belongs. The terms varus, valgus, anterior, posterior, proximal, distal, medial, lateral, sagittal, coronal, and transverse are used herein with their conventional medical/anatomical meaning as defined, for example, in Dorland's Illustrated Medical Dictionary. The term laxity shall mean slackness or displacement in the rotational motion of the knee joint. The term restraint shall mean restriction or confinement in the rotational motion of the knee joint. Valgus-varus restraint shall mean rotational or tilting restriction or confinement in the coronal plane. Medial-lateral restraint shall mean rotational restriction or confinement in the transverse plane. The term "change in restraint δR" shall mean the increase or decrease in valgus-varus restraint or medial-lateral restraint or degree of rotation of the prosthesis in the sagittal plane. Because the components rotate, the orientation of various features is described with reference to the configuration in full flexion as show in Fig. 1.

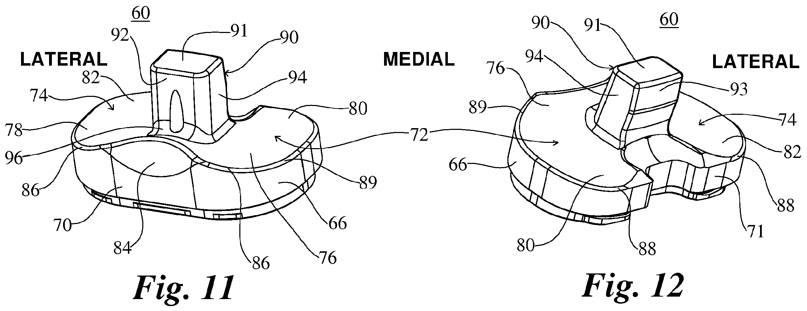

A revision knee prosthesis is illustrated in Figs. 1-23 and is designated generally by reference numeral 10. The prosthesis 10 includes a femoral component 20, constructed and designed to be fixed to the distal end of a resected femur, and a tibial component 52, constructed and designed to be fixed to the proximal end of a resected tibia. The components 20, 52 can be fixed to the femur and tibia, respectively, using conventional methods after conventional femoral and tibial revision resection. The tibial component 52 has a symmetrical design that can be used on either the left or right knee; however, the femoral component is asymmetrical and is illustrated in Figs. 1-23 for installation on the left knee. A mirror image of the femoral component 20 will be used for installation on the opposite knee.

The femoral component 20 has a medial condylar portion or condyle 22, a lateral condylar portion or condyle 24, a patellar flange portion or flange 26, which bridges the anterior ends 28, 30 of the medial 22 and lateral 24 condyles, respectively, and a cam 47 that bridges and connects the posterior ends 29, 31 of the medial 22 and lateral 24 condyles. The medial 22 and lateral 24 condyles are arranged in substantially parallel relationship to each other and define an intercondylar notch 32 there between. As the prosthesis flexes, different sections of the curved condylar portions engage and articulate with the tibial component 52.

The patellar flange 26 includes a patellar groove 42, which is flanked by a medial trochlear surface 44 and a lateral 46 trochlear surface. The patellar flange is designed to articulate with either the natural patella or a patellar component. The patellar flange transitions smoothly with the condyles 22, 24. The patellar flange is constructed by approximating the geometry of the distal anterior surface of a natural femur. As a result, the patellar flange has natural tracking of the prosthetic or natural patella.

Each condyle 22, 24 generally comprises an anterior 34, 36 and posterior 38, 40 surface, which blend smoothly with each other without any abrupt transition. In a preferred example shown in Figs. 1-23, the anterior and posterior surfaces resemble toroidal sections, with each surface having its radius about the major axis (major radius) substantially in the sagittal plane. In general, the major radius of curvature of the condyles 22, 24 varies from front to back to mimic anatomic femoral rollback during high degrees of flexion. For example, the major radius of the anterior surface 34 is preferably larger than the major radius of the posterior surface 38. Additionally, the major radius of the anterior and posterior surfaces may also reduce proceeding posteriorly. For example, in the arrangement shown in Figs. 1-23, the anterior surfaces 34, 38 have gradually reducing major radii while the posterior surfaces 36, 40 have rapidly reducing major radii proceeding posteriorly. Proximate the posterior ends 29, 31 of the condyles 22, 24, the major radius is preferably greatly reduced to allow the prosthesis to flex preferably greater than 100 degrees, and more preferably more than 130 degrees. Further, the small radii of the ends 29, 31 prevent edge loading of the condyles 29, 31 while maintaining contact between the components on the tibial liner 60.

The condyles 22, 24 have a radius about the minor axis (minor axis) in the coronal plane. In a preferred embodiment, the condyles have a constant radius of curvature in the coronal plane. However, the condylar surfaces could have a more complex geometry wherein the radius of curvature in the coronal plane can vary. In particular, the posterior condylar surfaces can be designed with varying radii in all three planes.

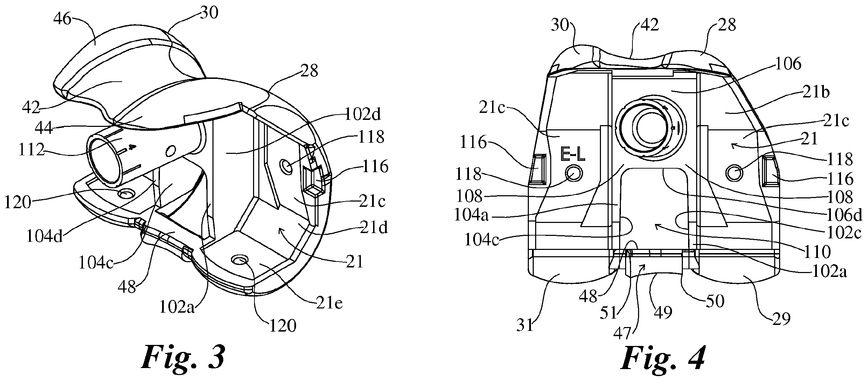

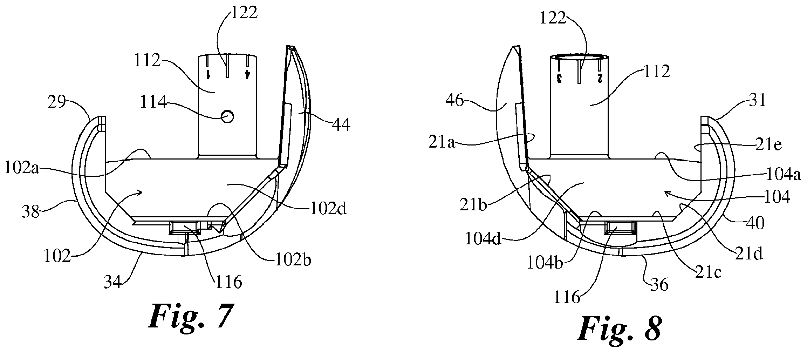

The interior boundaries of the medial 22 and lateral 24 condyles define an intercondylar notch 32 there between. A guide box 100 is centrally fixed to the proximal mounting surface 21 of the femoral component 20 and straddles the intercondylar notch 32. The guide box 100 is formed from a medial 102 and lateral 104 web that extend proximally in generally-parallel sagittal planes along the interior edge of each condylar portion 22, 24. Referring to Figs. 7-8, each web 102, 104 has a straight top edge 102a, 104a, and an irregularly-shaped bottom edge 102b, 104b that is integrally formed with the irregularly-shaped proximal mounting surface 21 at the interior edge of each condylar portion 22, 24, respectively. While the height of the webs will vary depending on the size of the prosthesis, the height of the webs should be great enough to provide the desired restraint when engaged with the central post 90 as described below. In the example shown in Figs. 1-22, the webs are about 13 to 20 mm high. As best seen in Fig. 4, a bridge plate 106 connects the upper edges 102a, 104a of the webs 102, 104. The bridge plate 106 extends in a transverse plane from the anterior mounting surface to an intermediate point 108 on the upper edge of each web 102, 104. The posterior edge 106d, cam 47, and upper edges 102a, 104a of the webs 102, 104 define a box guide slot 110 in which the central post 90 translates and rotates as described below.

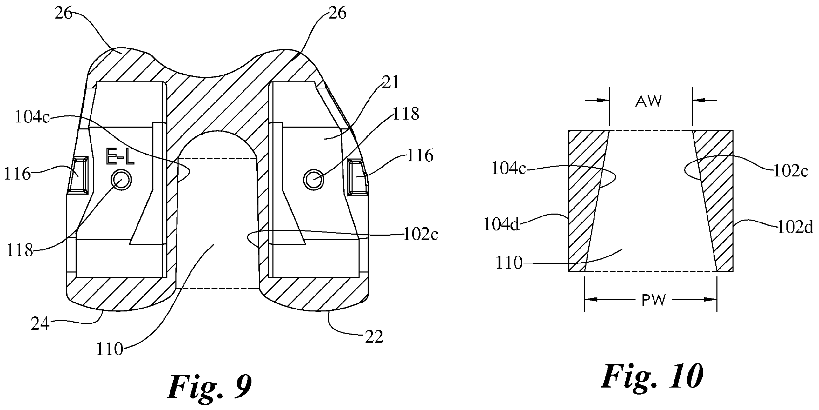

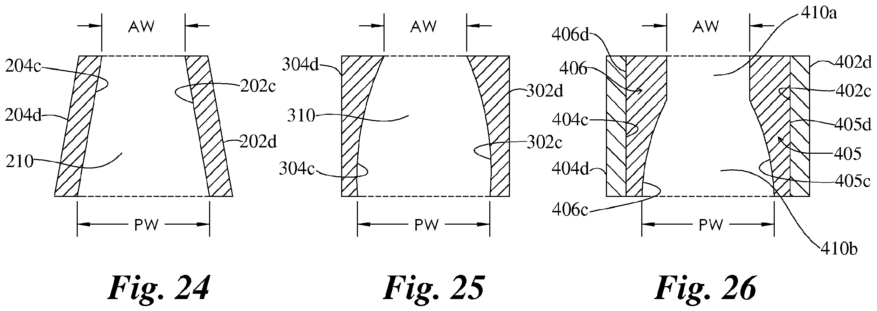

Referring to Figs. 4, 9 and 10, the guide box slot 110 has a generally trapezoidal shape. As best seen in the enlarged illustration of Fig. 10, the slot 110 has an anterior end width "AW" and a posterior end width "PB" that is wider than "AW." While shown exaggerated in Fig. 10, the difference between "AW" and "PW" is preferably about 1 mm but may smaller or larger depending on the desired performance characteristics of the prosthesis. In a preferred embodiment, the medial 102 and lateral 104 webs have reducing thickness proceeding posteriorly. In this example, the exterior surfaces 102d, 104d of the webs are parallel to one another while the interior surfaces 102c, 104c are skew, which creates the trapezoidal interior guide slot. In another example shown in Fig. 24, similar functionality could be achieved with web walls 202, 204 having a uniform thickness. However, in this example, the interior walls 202c, 204c and exterior walls 202d, 204d are skew.

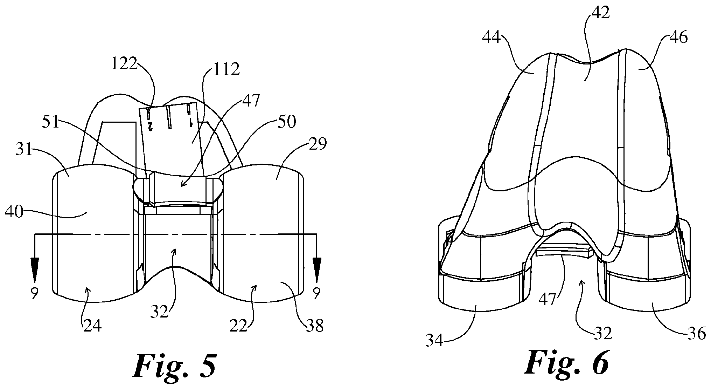

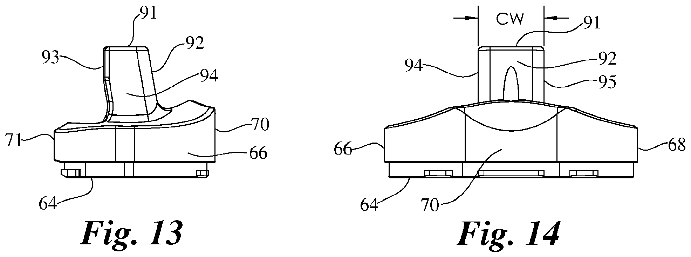

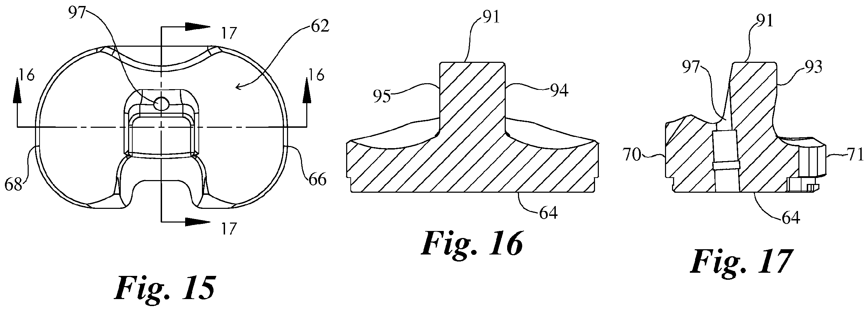

As best seen in Fig. 5 and 14, a cam 47 bridges and connects the posterior ends 29, 31 of the medial 22 and lateral 24 condyles. Referring to Fig. 16, the cam 47 has a generally flat back surface 48 and a curved front bearing surface 49. The back surface 48 is oriented coplanar with the posterior portion of the mounting (proximal) surface 21 of the condyles 22, 24 and is flat to abut the resected surface of the distal femur. As best seen in Fig. 5, the radius of curvature of the bearing surface 49 is larger at the lateral end 51 than the medial end 50. As described in greater detail below, the cam 47 engages the central post 90 on the tibial bearing liner 60 to provide stability and tibial rotation as the knee is flexed.

A boss 112 is fixed to the proximal surface of the bridge plate 106. The boss 112 has a generally cylindrical shape and is dimensioned to receive an intermedullary mounting stem 9 shown in Fig. 1, which helps secure the femoral component using known revision surgery techniques. A radial bore 114 extends through the wall of the boss 112. As best seen in Fig. 5, the boss extends at a slight angle relative to the bridge plate 106. The angle approximates the angle of the femoral intermedullary canal relative to the ground. Once the stem is inserted into the boss 112, a set screw or pin is inserted through the bore 114 and into the stem to lock the stem in the bore 114. The boss 112 also includes a plurality of alignment indicators 122 for properly orienting the femoral component 20 on the femoral stem 8.

Referring to Figs. 3 and 4, the proximal mounting surface 21 comprises discrete surface portions 21a-e proceeding from the patellar flange portion 26 to the posterior end of the condylar portion 29, 31. The discrete mounting surfaces engage complimenting surfaces on the resected femur and are secured thereto with cement. The intermediate mounting surface 21c and posterior mounting surface 21e include threaded bores 118, 120, respectively, which engage threaded fasteners used to affix augments (not shown), if needed. The intermediate mounting surface 21c also includes medial and lateral notches 116, which assist removal of the femoral component during sizing and/or replacement. The notches 116 also act as cement pockets once the correctly-sized component is permanently affixed to the resected femur.

Referring to Fig. 1, the tibial component 52 generally comprises a tibial platform 54 and a liner 60. The tibial platform 54 has a base plate 55, which engages the distal surface 64 of the liner 60, and a stabilizing keel 56, which is inserted into the medullary canal of the tibia. The underside or distal surface of the base plate 55 may have a textured, roughened surface that allows cement interdigitation during installation on the tibia.

The liner 60 has a proximal bearing surface 62, which articulates with the femoral component 20, and a distal surface 64, which abuts and is fixed to the tibial platform 52. The tibial component 50 also has a medial side 66, a lateral side 68, an anterior side 70, and a posterior side 71. The tibial component is generally symmetrical about a central sagittal axis running anterior to posterior.

A medial concavity 72 and a lateral concavity 74 are formed in the medial and lateral sides of the proximal surface 62, respectively. The medial 72 and lateral 74 concavities engage the medial 22 and lateral 24 condyles of the femoral component 20 as the components articulate relative to one another. In general, the concavities 72, 74 are shallower than the depth of the femoral condyles 22, 24.

Each concavity 72, 74 generally comprises an anterior 76, 78 and posterior 80, 82 surface, respectively, that resemble toroidal sections, which blend together at an intermediate boundary. In a preferred embodiment, the anterior surfaces 76, 78 have a major radius of curvature oriented substantially in the sagittal plane. Preferably, the posterior surfaces 80, 82 have a major radius of curvature oriented substantially in the transverse plane. The posterior concavities 80, 82 curve inwardly toward the sagittal central axis and sweep posteriorly and toward the central sagittal axis. The anterior 76, 78 and posterior 80, 82 surfaces have the same constant radius of curvature in the coronal plane and share the same tangent intersection in the coronal plane. The coronal curvature of the posterior surface is maintained as it turns toward the central sagittal axis. This construction allows the tibia to rotate about its longitudinal axis and translate posteriorly as the knee flexes.

The anterior and posterior concavities may have a raised periphery at the anterior 86 and posterior 88 ends to contain and prevent dislocation of the femur from the tibia. The raised periphery also provides stability to the knee during flexion. The anterior concavities may have lateral elevations 89, which contain the condyles 22, 24 so that the tibial component has little laxity during initial flexion and prevent tibial axial rotation. In contrast, the posterior concavities are designed without constraining lateral elevations and are designed to enable tibial axial rotation.

In the example shown in Figs. 1-23, the tibial component 60 includes a central post 90, which has a proximal surface 91, anterior surface 92, posterior surface 93, medial side surface 94, and lateral side surface 95. The anterior surface 92 of the central post 90 is tapered at an angle relative to the distal surface 64 to minimize impingement of the patella or a patellar implant in deep flexion. As best seen in Fig. 14, the medial 94 and lateral 95 sides of the post 90 are parallel and have a constant medial-lateral width "MLW". In a preferred example, the medial-lateral width is slightly less than the anterior width "AW" of the box guide slot. To maximize its restraining performance, the height of the post 90 should approximate but be slightly less than the height of webs 102, 104; otherwise, the post 90 will interfere with the bridge plate 106 when the knee is positioned near full extension.

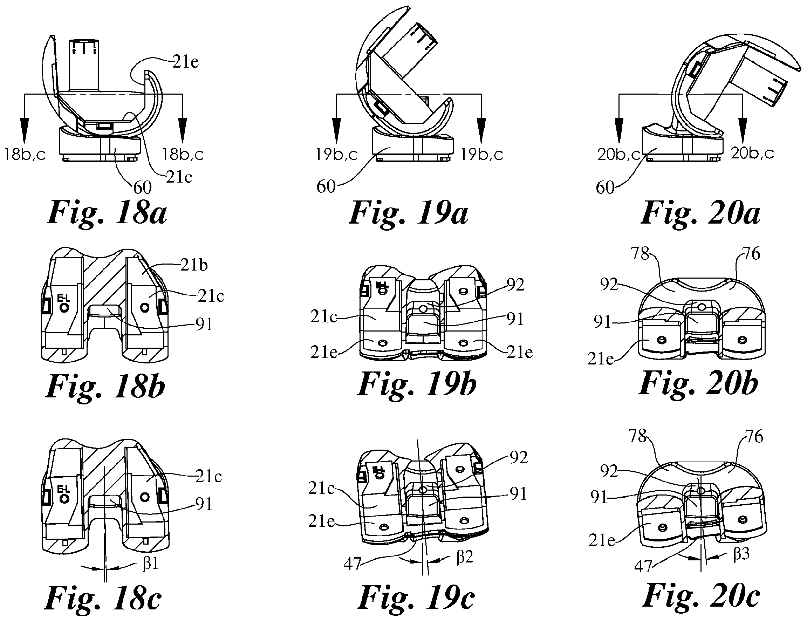

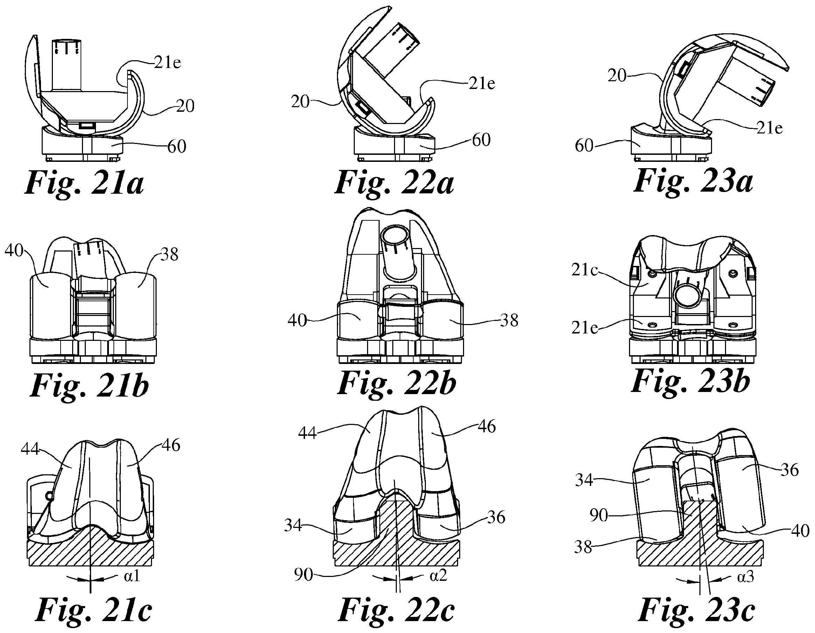

The relative motion path of the femoral and tibial components is illustrated in Figs. 18-23. Like the natural knee, the anterior surfaces 34, 38 of the condyles 22, 24 contact the tibial component in the range of full extension (Figs. 18 and 21) to an intermediate position in partial flexion (Figs. 19 and 22), and then to full flexion (Figs. 20 and 23). As the femoral component rotates, the central post 90 translates within the box guide slot 110.

In full extension, the central post 90 is positioned in the anterior end portion of the guide slot as best seen in Fig 18b. In this position, the prosthesis has maximum medial-lateral restraint and valgus-varus restraint since the medial-lateral width "CW" of the central post 90 is only slightly less than the anterior, interior width "AW" of the guide slot 110. In a preferred example, the difference "AW" minus "CW" is about 0.1 to 0.25 mm. Therefore, the femoral component has very little transverse rotational laxity, represented by alpha 1(α1) in Fig. 18c. In a preferred example, alpha (α1) is about 1degree. The femoral component also has very little medial-lateral rotational laxity, represented as beta 1 (β1) in Fig. 21c. In a preferred example, beta 1 (β1) is about 2 degrees.

As the femoral component rotates to an intermediate position show in Figs. 19a and Fig. 22a, the central post 90 moves within the guide slot 110 to a position intermediate the anterior and posterior end portions of the guide slot as best seen in Fig 18b. In this intermediate position, the prosthesis has decreased medial-lateral restraint and decreased valgus-varus restraint compared to full extension since the gap between the sides of the central post 90 and the inside surfaces 102c, 104c of the medial 102 and lateral 104 webs is increased. The gap is increased since the interior width of the slot 110 is wider in the intermediate region of the guide slot than the anterior width AW, while the medial lateral width "CW" of the central post 90 is constant. Therefore, the femoral component has increased transverse rotational laxity alpha 2 (α2) compared to α1 as illustrated in Fig. 19c. The femoral component also has increased medial-lateral rotational laxity beta 2 (β2) compared to β1 as illustrated in Fig. 22c.

As the femoral components rotates to a position in full flexion shown in Figs. 20 and 23, the asymmetric cam 47 articulates with the tibial post 90 and causes the femoral component to translate posteriorly on the tibial component 52 as best seen in Figs. 20 and 23. In the preferred example, posterior translation of the femoral component is limited to about 1-2 millimeters. In full flexion, the central post 90 is positioned in the posterior end portion of the guide slot as best seen in Fig 20b. In this position, the prosthesis has minimum medial-lateral restraint and minimum valgus-varus restraint since the gap between the sides of the central post 90 and the inside surfaces 102c, 104c of the medial 102 and lateral 104 webs is increased even greater compared to the intermediate position. The gap is increased since the interior width of the guide slot at the posterior end "PW" is maximized, while the medial-lateral width "CW" of the central post 90 is constant. In a preferred example, the difference "PC" minus "CW" is about 0.5 to 1 mm. Therefore, the femoral component has increased transverse rotational laxity alpha 3 (α3) compared to α2 as illustrated in Fig. 20c. In a preferred example, α3 is less than about 4 degrees. The femoral component also has increased medial-lateral rotational laxity beta 3 (β3) compared to β2 as illustrated in Fig. 23c. In a preferred example, β3 is less than about 7 degrees.

In a similar manner, as the femoral component rotates from full flexion, or any intermediate position in flexion, back to full extension, valgus-varus restraint and medial-lateral restraint gradually increase. As the femoral component returns to full extension, the gap between the central post 90 and the guide slot 110 reduces to a minimum, thereby further restraining the knee.

As described above, the unique design of the prosthesis enables desirable maximum valgus-varus and medial-lateral rotational restraint in full flexion. The unique design also provides progressively decreasing and increasing restraint as the knee flexes. Because of this unique design, the curved bearing surface 49 of the cam 47 and the geometry of the bearing surfaces cause the tibia to rotate axially as the knee flexes. In a preferred example, tibial rotation is enabled up to at least about 5 degrees, preferably up to at least about 6 degrees, more preferably up to about 7 degrees. However, the cam and articulating surfaces could be designed to enable greater tibial axial rotation if desired.

It should be appreciate by those skilled in the art that the prosthesis 10 can be provided in a variety of sizes to accommodate patients of varying sizes. Therefore, the dimensions described above with respect to certain components will obviously vary.

Furthermore, the prosthesis can be provided in a wide variety of performance characteristics without departing from the scope of the invention. For example, the prosthesis 10 can be custom designed with high restraint in full flexion and a small δR or large δR. Similarly, the prosthesis 10 can be designed with less restraint in full flexion and a small δR or large δR. In each case, however, the relative level of restraint will be change over the range of flexion of the prosthesis 10.

In the examples shown in Figs. 10 and 24, the planar profile of the inner surfaces of the guide slot create a constant change (delta) in restraint, δR, on the femoral component as it rotates. In embodiments of the invention, the inside surfaces of the medial and lateral webs have non-planar or irregular shapes that create a variety of δR profiles. For example, the curved inside surface profile shown in Fig. 25 creates a variable δR over the full range of flexion.

Furthermore, to create even more complex δR profiles, the inner surfaces of the medial and lateral webs could be fitted with separate cam surfaces 405, 406 such as shown in Fig. 26. The exterior surfaces 405d, 406d of the cams abut the interior surfaces 402c, 404c of the medial and lateral webs. In the example shown in Fig. 26, the inner surfaces 405c, 406c of the cams create zero δR in the first region 410a of the slot and a variable δR in the second region 410b of the slot. The regions 410a and 410b correspond to defined ranges of flexion. For example, the first region 410a corresponds to a flexion range of 0 degrees to X degrees, *i.e*., full extension to a predefined intermediate angular position X. Within the first flexion range, the prosthesis would have a constant, predefined level of restraint. The second region 410b corresponds to a flexion range of X degrees to Y degrees, *i.e.,* the intermediate position to the maximum angular position Y. Within the second flexion range, the level of restraint decreases and increases at a non-constant rate as the knee cyclically flexes from the X degrees to Y degrees and then back to X degrees. The values of X and Y can vary.

Even more complex cams can be fitted to the inner surfaces of the medial and lateral webs, which would create multiple, discrete δR profiles over multiple, discrete ranges of flexion. Many other useful surface profiles should be readily apparent to those skilled in the art.

The femoral component and tibial component may have various surface profiles and may be constructed in various manners and from various materials such as those described in U.S. patent application no. 12/388,125 entitled Total Knee Replacement Prosthesis with High order NURBS Surfaces, filed 2/18/2009, and U.S. patent application no. 12/388,182 entitled Total Knee Replacement Prosthesis, filed 2/1 8/2009. For example, the femoral component 20 and the tibial platform 54 may be machined, cast, forged or otherwise constructed as a one-piece integral unit from a medical grade, physiologically acceptable metal such as cobalt chromium alloy, stainless steel, titanium, titanium alloy or nickel cobalt alloy.

The tibial liner may also be constructed in various manners and from various materials. For example, the tibial liner may be machined, molded or otherwise constructed as a one-piece, integral unit out of a medical grade, physiologically acceptable polymeric materials such as any polyolefin, including high-density polyethylene, low-density polyethylene, linear-low-density polyethylene, ultra-high molecular weight polyethylene (UHMWPE), or mixtures thereof. Polymeric materials, as used herein, also include polyethylene of various forms, for example, resin powder, flakes, particles, powder, or a mixture thereof, or a consolidated form derived from any of the above. Ultra-high molecular weight polyethylene (UHMWPE) refers to linear, non-branched chains of ethylene having initial average molecular weights in excess of about 500,000, preferably above about 1,000,000, and more preferably above about 2,000,000. Often the molecular weights can reach about 8,000,000 or more. The material can be treated, for example, by radiation, chemistry, or other technology to alter its wear properties and/or strength or hardness. Initial average molecular weight means the average molecular weight of the UHMWPE starting material, prior to any irradiation.

It is to be understood that the description, specific examples and data, while indicating exemplary embodiments, are given by way of illustration and are not intended to limit the present invention, the scope of which is defined by the appended claims. Various changes and modifications within the present invention will become apparent to the skilled artisan from the discussion, disclosure and data contained herein, and thus are considered part of the invention.

## Claims

1. A knee revision prosthesis (10) having anterior, posterior, lateral, medial, distal and proximal sides and sagittal, coronal and transverse planes, comprising:
a) a femoral component (20) that connects to the distal end of a resected femur, said femoral component (20) including:
i) medial and lateral condyles (22, 24) having distal, articulating surfaces and proximal mounting surfaces (21a-e);
ii) a patellar flange (26) having a patellar articulating surface that bridges the anterior ends (28, 30) of the femoral articulating surfaces;
b) a tibial component (52) that connects to the proximal end of a resected tibia, said tibial component (52) including a proximal bearing surface (62) with medial and lateral concavities (72, 74) that articulate with said medial and lateral condyles (22, 24);
c) progressive constraint means for providing progressively increasing and progressively decreasing rotational constraint of the femoral component (20) in the coronal and transverse planes as the femoral component (20) rotates in the sagittal plane between full extension, to a position in flexion, and then back to full extension;
**characterized in that** the progressive constraint means provides rotational constraint of the femoral component (20) in the coronal and transverse planes at a non-constant rate of change as the femoral component (20) rotates in the sagittal plane between full extension, to a position in flexion, and then back to full extension.

2. The knee revision prosthesis (10) recited in claim 1 including
i) a guide box (100) fixed to the femoral mounting surfaces (21a-e) intermediate said condyles (22, 24); and
ii) a central post (90) fixed intermediate said tibial concavities (72, 74) which articulates within said guide box (100) and constrains both rotational and translational movement of the femoral and tibial components (20, 52) relative to one another in the coronal plane and transverse plane;
wherein said post (90) and guide box (100) are constructed and arranged to provide progressively decreasing and then progressively increasing rotational constraint of the femoral component (20) in the coronal and transverse planes as the femoral component (20) rotates in the sagittal plane between full extension to a position in flexion, and then back to full flexion, respectively.

3. The knee revision prosthesis (10) recited in claim 1, wherein the femoral component (20) is restrained from rotating more than about 2 degrees in the coronal plane when the prosthesis (10) is positioned in full extension and wherein the femoral component (20) is restrained from rotating-more than about 7 degrees in the coronal plane when the prosthesis (10) is positioned in full flexion.

4. The knee revision prosthesis (10) recited in claim 1, wherein the femoral component (20) is restrained from rotating more than about 1 degree in the transverse plane when the prosthesis (10) is positioned in full extension and wherein the femoral component (20) is restrained from rotating more than about 4 degrees in the transverse plane when the prosthesis (10) is positioned in full flexion.

5. The knee revision prosthesis (10) recited in claim 1, wherein the change in restraint δR on the femoral component (20) is variable over the full range of flexion.

6. The knee revision prosthesis (10) recited in claim 1, wherein the change in restraint δR on the femoral component (20) has a first profile over a first range of flexion, and has a second profile over a second range of flexion.

7. The knee revision prosthesis (10) recited in claim 2, wherein said guide box (100) has opposed side walls (202, 204) with interior surfaces (102c, 104c) having a minimum interior width between them at an anterior end AW and a maximum interior width, and said post (90) has opposed exterior surfaces that engage the interior surfaces (102c, 104c) of said guide box(100).

8. The knee revision prosthesis (10) recited in claim 7, wherein the guide box side walls (202, 204) have exterior surfaces (102d, 104d) that are parallel to one another and interior side walls (202c, 204c) that are skew.

9. The knee revision prosthesis (10) recited in claim 7, wherein said guide box interior surfaces (102c, 104c) have an irregular surface profile.

10. The knee revision prosthesis (10) recited in claim 7, including two opposed cams (47), one fixed to each of the interior surfaces (102c, 104c) of said side walls (202, 204), said cams (47) having interior surfaces (405c, 406c) that engage the exterior surfaces of said post (90).

11. The knee revision prosthesis (10) recited in claim 10, wherein the interior surfaces (405c, 406c) of said cams (47) have a minimum interior width between them at an anterior end AW and a maximum interior width and the post (90) has exterior surfaces that have a constant width CW between them that is less than AW.

12. The knee revision prosthesis (10) recited in claim 11, wherein the interior surfaces (405c, 406c) of said cams (47) have an irregular surface profile.

13. The knee revision prosthesis (10) recited in claim 2, wherein said femoral component (20) includes a cam (47) connecting the posterior ends (29, 31) of the condyles (22, 24), and said tibial component (52) includes a central post (90) intermediate said concavities (72, 74), wherein anterior and posterior translation of the femoral component (20) relative to the tibial component (52) is controlled by said cam (47) and central post (90); and wherein rotation of the tibia about its longitudinal axis is controlled by said cam (47) and central post (90).

14. The knee revision prosthesis (10) recited in claim 13, wherein posterior translation is about 1-2 millimeters after the prosthesis is (10) fully flexed.

## Patentansprüche

1. Eine Knierevisionsprothese (10) mit anterioren, posterioren, lateralen, medialen, distalen und proximalen Seiten und sagittalen, koronaren und transversalen Ebenen umfassend:
a) eine Femur-Komponente (20), die mit dem distalen Ende eines resezierten Femurs verbunden ist, wobei besagte Femur-Komponente (20) Folgendes umfasst:
i) mediale und laterale Kondylen (22, 24) mit distalen Artikulationsflächen und proximalen Befestigungsflächen (21a-e);
ii) einen Patella-Flansch (26) mit einer Patella-Artikulationsfläche, welche die anterioren Enden (28, 30) der Femur-Artikulationsflächen überbrückt;
b) eine Tibia-Komponente (52), welche mit dem proximalen Ende einer resezierten Tibia verbunden ist, wobei besagte Tibia-Komponente (52) eine proximale Tragfläche (62) mit medialen und lateralen Konkavitäten (72, 74) umfasst, welche mit besagten medialen und lateralen Kondylen (22, 24) artikulieren;
c) ein progressives Beschränkungmittel zur Bereistellung einer progressiv zunehmenden und einer progressiv abnehmenden Rotationsbeschränkung der Femur-Komponente (20) in der koronaren und transversalen Ebene, während die Femur-Komponente (20) in der sagittalen Ebene zwischen voller Extension, in eine Position in Flexion und dann wieder in die volle Extension rotiert;
**dadurch gekennzeichnet, dass** das progressive Beschränkungsmittel eine Rotationsbeschränkung der Femur-Komponente (20) in der koronaren und transversalen Ebene bei einer nicht konstanten Veränderungsrate bereitstellt, während die Femur-Komponente (20) in der sagittalen Ebene zwischen voller Extension, in eine Position in Flexion und dann wieder in die volle Extension rotiert.

2. Die Knierevisionsprothese (10) gemäß Anspruch 1 umfassend
(i) einen Führungskasten (100) befestigt an den Femur-Befestigungsflächen (21a-e) zwischen besagten Kondylen (22, 24); und
(ii) einen zentralen Pfosten (90) befestigt zwischen besagten Tibia-Konkavitäten (72, 74), welcher innerhalb des besagten Führungskastens (100) artikuliert und sowohl die rotationale als auch die translationale Bewegung der Femur- und Tibia-Komponenten (20, 52) in Relation zueinander in der koronaren Ebene und der transversalen Ebene beschränkt;
wobei der besagte Pfosten (90) und der besagte Führungskasten (100) so konstruiert und angeordnet sind, dass sie eine progressiv abnehmende und dann progressiv zunehmende rotationale Beschränkung der Femur-Komponente (20) in der koronaren und der transversalen Ebene bereitstellen, während die Femur-Komponente (20) in der sagittalen Ebene respektive zwischen voller Extension, in eine Position in Flexion und dann wieder in die volle Extension rotiert.

3. Die Knierevisionsprothese (10) gemäß Anspruch 1, wobei die Femur-Komponente (20) an der Rotation um mehr als etwa 2 Grad in der koronaren Ebene gehindert wird, wenn die Prothese (10) in voller Extension positioniert ist, und wobei die Femur-Komponente (20) an der Rotation um mehr als etwa 7 Grad in der koronaren Ebene gehindert wird, wenn die Prothese (10) in voller Flexion positioniert ist.

4. Die Knierevisionsprothese (10) gemäß Anspruch 1, wobei die Femur-Komponente (20) an der Rotation um mehr als etwa 1 Grad in der transversalen Ebene gehindert wird, wenn die Prothese (10) in voller Extension positioniert ist, und wobei die Femur-Komponente (20) an der Rotation um mehr als etwa 4 Grad in der transversalen Ebene gehindert wird, wenn die Prothese (10) in voller Flexion positioniert ist.

5. Die Knierevisionsprothese (10) gemäß Anspruch 1, wobei die Veränderung der Beschränkung (δR) an der Femur-Komponente (20) über den gesamten Flexionsbereich hinweg variabel ist.

6. Die Knierevisionsprothese (10) gemäß Anspruch 1, wobei die Veränderung der Beschränkung (δR) an der Femur-Komponente (20) ein erstes Profil über einen ersten Flexionsbereich hinweg und ein zweites Profil über einen zweiten Flexionsbereich hinweg aufweist.

7. Die Knierevisionsprothese (10) gemäß Anspruch 2, wobei besagter Führungskasten (100) gegenüberliegende Seitenwände (202, 204) mit Innenflächen (102c, 104c) aufweist, zwischen denen eine Mindestinnenbreite an einem anterioren Ende (AW) und eine Höchstinnenbreite besteht, und wobei besagter Pfosten (90) gegenüberliegende Außenflächen aufweist, die in die Innenflächen (102c, 104c) des besagten Führungskastens (100) greifen.

8. Die Knierevisionsprothese (10) gemäß Anspruch 7, wobei besagte Führungskastenwände (202, 204) Außenflächen (102d, 104d) aufweisen, welche parallel zueinander sind, und Innenseitenwände (202c, 204c), die schräg sind.

9. Die Knierevisionsprothese (10) gemäß Anspruch 7, wobei besagte Führungskasteninnenflächen (102c, 104c) ein unregelmäßiges Oberflächenprofil aufweisen.

10. Die Knierevisionsprothese (10) gemäß Anspruch 7 umfassend zwei gegenüberliegende Nocken (47), eine an jeder der Innenflächen (102c, 104c) der besagten Seitenwände (202, 204) befestigt, wobei besagte Nocken (47) Innenflächen (405c, 406c) haben, die in die Außenflächen des besagten Pfostens (90) greifen.

11. Die Knierevisionsprothese (10) gemäß Anspruch 10, wobei zwischen den Innenflächen (405c, 406c) der besagten Nocken (47) eine Mindestinnenbreite an einem anterioren Ende (AW) und eine Höchstinnenbreite besteht, und wobei der Pfosten (90) Außenflächen hat, zwischen denen eine konstante Breite (CW) besteht, die kleiner als AW ist.

12. Die Knierevisionsprothese (10) gemäß Anspruch 11, wobei die Innenflächen (405c, 406c) der besagten Nocken (47) ein unregelmäßiges Oberflächenprofil aufweisen.

13. Die Knierevisionsprothese (10) gemäß Anspruch 2, wobei besagte Femur-Komponente (20) einen Nocken (47) aufweist, welcher die posterioren Enden (29, 31) der Kondylen (22, 24) verbindet, und wobei besagte Tibia-Komponente (52) einen zentralen Pfosten (90) zwischen besagten Konkavitäten (72, 74) umfasst, wobei die anteriore und posteriore Translation der Femur-Komponente (20) in Relation zur Tibia-Komponente (52) durch besagten Nocken (47) und zentralen Pfosten (90) kontrolliert wird; und wobei die Rotation der Tibia um ihre Längsachse durch besagten Nocken (47) und zentralen Pfosten (90) kontrolliert wird.

14. Die Knierevisionsprothese (10) gemäß Anspruch 13, wobei die posteriore Translation nach der vollen Flexion der Prothese (10) etwa 1-2 Millimeter beträgt.

## Revendications

1. Une prothèse de révision du genou (10) disposant des parois antérieures, postérieures, latérales, médiales, distale et proximales et des plans sagittal, coronal et transversal comprenant :
a) un composant fémoral (20) qui se relie à une extrémité distale d'un fémur réséqué, dit composant fémoral (20) y compris :
i) les condyles médial et latéral (22, 24) ayant des surfaces distales, des surfaces d'articulations et des surfaces de montage proximales (21a-e) ;
ii) une bride rotulienne (26) ayant une surface d'articulation rotulienne qui relie les extrémités antérieures (28, 30) des surfaces d'articulations fémorales ;
b) un composant tibial (52) qui relie l'extrémité proximale d'un tibia réséqué, dit composant tibial (52) y compris une surface d'appui distale (62) avec des concavités médiale et latérale (72, 74) qui s'articule avec les condyles médial et latéral (22, 24) ;
c) des moyens de contrainte progressive pour fournir une contrainte progressivement croissante et progressivement décroissante du composant fémoral (20) dans les plans coronal et transversal lors de la rotation du composant fémoral (20) dans le plan sagittal entre une extension totale, une flexion et ensuite une extension totale.
**caractérisé par** les moyens de contrainte progressive fournissent une contrainte de rotation du composant fémoral (20) dans les plans coronal et transversal à un taux de changement variable lors de la rotation du composant fémoral (20) dans le plan sagittal entre une extension totale, une flexion et ensuite une extension totale.

2. La prothèse de révision du genou (10) mentionnée dans la revendication 1 y compris
i) une boîte de guidage (100) fixée aux surfaces de montage fémorales intermédiaires (21a-e) desdits condyles (22, 24) ; et
ii) un montant central (90) fixé aux concavités tibiales intermédiaires (72, 74) qui s'articule dans ladite boîte de guidage (100) et retient le mouvement rotationnel et translationnel des composants fémoral et tibial (20, 52) l'un par rapport à l'autre dans le plan coronal et le plan transversal ;
alors que ledit montant (90) et la boîte de guidage (100) sont construits et disposés afin de fournir une contrainte rotationnelle progressivement décroissante et ensuite progressivement croissante du composant fémoral (20)dans les plans coronal et transversal lorsque le composant fémoral (20) tourne dans le plan sagittal respectivement à partir d'une extension totale pour une position en flexion et une flexion totale à nouveau.

3. La prothèse de révision du genou (10) mentionnée dans la revendication 1, où la rotation du composant fémoral (20) est retenue de plus de 2 degrés environ dans le plan coronal lorsque la prothèse (10) est placée en extension totale et où la rotation du composant fémoral (20) est retenue de plus de 7 degrés environ dans le plan coronal lorsque la prothèse (10) est placée en flexion totale.

4. La prothèse de révision du genou (10) mentionnée dans la revendication 1, où la rotation du composant fémoral (20) est retenue de plus de 1 degré environ dans le plan transversal lorsque la prothèse (10) est placée en extension totale et où la rotation du composant fémoral (20) est retenue de plus de 7 degrés environ dans le plan transversal lorsque la prothèse (10) est placée en flexion totale.

5. La prothèse de révision du genou (10) mentionnée dans la revendication 1, où le changement de restriction SR sur le composant fémoral (20) est variable sur toute l'amplitude de flexion.

6. La prothèse de révision du genou (10) mentionnée dans la revendication 1, où le changement de
restriction SR sur le composant fémoral a un premier profil sur une première amplitude de flexion et un deuxième profil sur une deuxième amplitude de flexion.

7. La prothèse de révision du genou (10) mentionnée dans la revendication 2, où ladite boîte de guidage (100) dispose de parois opposées (202, 204) avec des surfaces intérieures (102c, 104c) ayant une largeur interne minimum entre elles à une extrémité antérieure AW et une largeur intérieure maximale et ledit montant (90) dispose de surfaces extérieures opposées qui entrent en contact avec les surfaces intérieures (102c, 104c) de ladite boîte de guidage (100).

8. La prothèse de révision du genou (10) mentionnée dans
la revendication 1, où les parois de la boîte de guidage (202, 204) disposent de surfaces extérieures (102d, 104d)
parallèles les unes aux autres et les parois intérieures (202c,
204c) obliques.

9. La prothèse de révision du genou (10) mentionnée dans la revendication 7, où les surfaces de ladite boîte de guidage (102c, 104c) ont un profil de surface irrégulier.

10. La prothèse de révision du genou (10) mentionnée dans la revendication 7 y compris deux cames opposées (47), une fixée à chaque surface intérieure (102c, 104c) desdites parois (202, 204), lesdites cames (47) ayant des surfaces intérieures (405c, 406c) qui entrent en contact avec les surfaces extérieures dudit montant (90).

11. La prothèse de révision du genou (10) mentionnée dans la revendication 10, où les surfaces intérieures (405c, 406c) desdites cames (47) ont une largeur intérieure minimale entre elles à une extrémité antérieure AW et une largeur intérieure maximale et le montant (90) dispose de surfaces extérieures qui ont une largeur constante CW entre elles, inférieures à AW.

12. La prothèse de révision du genou (10) mentionnée dans la revendication 11,
où les surfaces intérieures (405c, 406c) des dites cames (47) ont un profil de surface irrégulier.

13. La prothèse de révision de genou (10) mentionnée dans la revendication 2, où ledit composant fémoral (20) inclut une came (47) reliant les extrémités postérieures (29, 31) des condyles (22, 24) et ledit composant tibial (52) inclut un montant central (90) intermédiaire desdites concavités (72, 74), où la translation antérieure et postérieure du composant fémoral (20) par rapport au composant tibial (52) est contrôlé par ladite came (47) et le montant central (90) ; et où la rotation du tibia autour de son axe longitudinal est contrôlée par ladite came (47) et le montant central (90).

14. La prothèse de révision du genou (10)) mentionnée dans la revendication 13, où la translation postérieure est d'environ 1 à 2 millimètres après la flexion totale de la prothèse (10).
